(19) Europäisches Patentamt European Patent Office Office européen des brevets

(11) **EP 2 362 786 B1**

(12) **EUROPEAN PATENT SPECIFICATION**

(45) Date of publication and mention
of the grant of the patent:
**29.03.2017 Bulletin 2017/13**

(51) Int Cl.:
**A61L 2/28** (2006.01)    **C12Q 1/22** (2006.01)

(86) International application number:
**PCT/US2009/063416**

(21) Application number: **09748679.9**

(22) Date of filing: **05.11.2009**

(87) International publication number:
**WO 2010/054095 (14.05.2010 Gazette 2010/19)**

(54) **PROCESS CHALLENGE DEVICE AND METHOD**

VERFAHRENS-CHALLENGE-VORRICHTUNG UND VERFAHREN

DISPOSITIF ET PROCÉDÉ DE TEST DE PROCESSUS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **06.11.2008 US 112041 P
06.11.2008 US 112149 P
06.11.2008 US 112071 P**

(43) Date of publication of application:
**07.09.2011 Bulletin 2011/36**

(73) Proprietor: **3M Innovative Properties Company
St. Paul, MN 55133-3427 (US)**

(72) Inventors:
• **FOLTZ, William, E.**
**Saint Paul, Minnesota 55133-3427 (US)**
• **HERRLEIN, Chad, M.**
**Saint Paul, Minnesota 55133-3427 (US)**
• **SCHMITZ, Joshua, M.**
**Minneapolis**
**Minnesota 55417 (US)**
• **ROBOLE, Barry, W.**
**Saint Paul, Minnesota 55133-3427 (US)**
• **WITCHER, Kelvin, J.**
**Saint Paul, Minnesota 55133-3427 (US)**
• **HOLT, Paul, N.**
**Saint Paul, Minnesota 55133-3427 (US)**

(74) Representative: **Vossius & Partner
Patentanwälte Rechtsanwälte mbB
Siebertstrasse 3
81675 München (DE)**

(56) References cited:
EP-A1- 1 308 174    EP-A2- 1 201 255
WO-A1-2009/137442    GB-A- 2 180 933
GB-A- 2 186 974    US-A- 3 981 683
US-A- 4 914 034    US-A1- 2001 006 610
US-A1- 2002 022 246    US-A1- 2003 157 588

**Description**

**Technical field**

The invention relates to a sterilization process challenge device comprising a first container comprising walls which define a first space within the first container; a process indicator within the first space; at least one sterilant access for a sterilant to enter the first space within the first container; wherein at least one of the walls which defines the first space is a flexible wall; and wherein the walls are impervious to the sterilant; a method of using the device.

BACKGROUND

A variety of products and articles, including medical instruments, must be sterilized prior to use to prevent bio-contamination of a sample, an organism, a wound site, or the like. A number of sterilization processes are used which involve contacting the product or article with a fluid sterilant, such as a gaseous sterilant. Examples of such sterilants include, for example, steam, ethylene oxide, hydrogen peroxide, and the like.

The products and articles are generally packaged such that the sterilant can pass through the packaging, but microorganisms cannot pass through. Even though the sterilant can pass, the packaging restricts the movement of the sterilant to the product or article. Moreover, some products and articles include spaces within them that can only be reached by the sterilant via a restricted path. For example, endoscopes often include a long, narrow channel through which the sterilant must pass in order to sterilize the endoscope. These and other forms of restrictions associated with products and articles to be sterilized must be taken into account when employing a sterilization process, so that all surfaces of the product or article are exposed to the sterilant for a time sufficient to cause sterilization.

Monitoring for sufficient sterilization is generally carried out by placing an appropriate sterilization indicator along with the product and/or article to be sterilized within a sterilization chamber. A variety of sterilization indicators, including biological and chemical indicators, are known and used for this purpose. However, to take into account the above described restrictions encountered in the various products and articles, the sterilization indicator has been placed in a challenge device which restricts the flow of sterilant to the indicator using a long tortuous path. While such devices have been useful, they have not always been convenient to use and/or they have not always provided a close correlation between an indication of complete sterilization and actual complete sterilization of the product or article.

As such, there continues to be an interest in and a need for challenge devices which are convenient to use and provide a more reliable correlation between the indication of complete sterilization and actual complete sterilization of a product or article.

US 2001/006610 A1 relates to sterilization indicator systems for determining the effectiveness of sterilization processes. The systems include a container that is open at one end and walls that are liquid impermeable and substantially gas-absorptive.

WO 2009/137442 A1 relates to a sterilization process challenge device. The device includes a solid body comprised of walls defining a space, and a flow restrictor associated with the solid body such that flow of sterilant into the space is restricted.

US 2002/022246 A1 relates to a sterilization process monitoring device. The device includes a container defining a first and a second space, and an antimicrobial source for providing an antimicrobial agent to the first space, wherein the second space is in communication with the antimicrobial source through the first space.

SUMMARY OF THE INVENTION

The present invention is defined by the features of the claims.

In one embodiment, the present invention provides a process challenge device comprising: a first container comprising walls which define a first space within the first container;

  a process indicator within the first space;
  at least one sterilant access for a sterilant to enter the first space within the first container; wherein at least one of the walls which defines the first space is a flexible wall; and wherein the walls are impervious to the sterilant; and
  a second container comprising:

    walls which define a second space within the second container; and
    at least one sterilant access for a sterilant to enter the second space; wherein the first container is within the second space; wherein at least one of the walls which defines the second space is a flexible wall; wherein the walls which define the second space are impervious to the sterilant; and wherein the second space has a volume which can vary as a pressure differential varies between the second space and outside of the second container.

The flexible wall allows the space within the container to change in volume as the pressure outside of the container changes. For example, when the pressure outside of the space is lowered, for example, when applying a vacuum, the volume may increase. In another example, when the pressure outside of the space is raised, for example, when applying a sterilant under pressure, the volume may decrease.

For certain embodiments, the process challenge device further comprises a heat-transfer modulating body adjacent the indicator. The heat-transfer modulating body may slow the rate at which the indicator comes to the temperature of a given sterilization process. For certain embodiments, the heat-transfer modulating body may also increase the time required for the sterilant to contact the indicator sufficiently to bring about an indication that sterilzation conditions have been achieved.

For certain embodiments, the sterilant access can be one or more openings, one or more ducts, one or more pressure-actuating valves, and a combination thereof. For certain embodiments, the sterilant access is sealed with a seal which can be removed when the device is put into use. For certain embodiments, at least two sterilant accesses are included. For certain embodiments, each of these sterilant accesses may be sealed with a removable seal. One, a portion, or all of the seals may be removed to prepare the device for use in a selected sterilization process.

For certain embodiments, the device further includes a second container, wherein the first container is within the second container. The second container comprises walls which define a second space within the second container; and at least one sterilant access for a sterilant to enter the second space; wherein the first container is within the second space; wherein at least one of the walls which defines the second space is a flexible wall; and wherein the walls which define the second space are impervious to the sterilant. Any of the sterilant access and volume features described herein for the first container may be included in the second container. For certain embodiments, this device provides the option of using the device with both first and second containers for a selected sterilization process, or removing the second container and using the resulting first container for another selected sterilization process. For certain embodiment, the first container may include removable seals on at least one but not all sterilant accesses included in the first container.

For certain embodiments, the device further includes at least one additional container, wherein the second container is within the at least one additional container. The at least one additional container comprises walls which define at least one additional space within the at least one additional container; and at least one sterilant access for a sterilant to enter the at least one additional space; wherein the second container is within the at least one additional space; wherein at least one of the walls which defines the at least one additional space is a flexible wall; and wherein the walls which define the at least one additional space are impervious to the sterilant. Any of the sterilant access and volume features described herein for the first container may be included in the at least one additional container. For certain embodiments, this device provides the option of using the device with both first, second, and the at least one additional containers for a selected sterilization process, or removing the at least one additional container and using the resulting device for another selected sterilization process. For certain embodiment, the second container may include removable seals on at least one but not all sterilant accesses included in the second container. For certain embodiments, the second container may also be removed and the resulting device used for another sterilization process as described above.

In another embodiment, there is provided a method of determining the effectiveness of a sterilization process, the method comprising:

> providing a process challenge device, including any one of the embodiments thereof described herein;
> positioning the process challenge device in a sterilization chamber;
> exposing the process challenge device to a sterilant at an elevated temperature; and
> determining whether or not the process indicator indicates that it has been exposed to sterilization process conditions effective for sterilizing an article.

For those embodiments wherein the sterilant accesses of the process challenge device are sealed with a removable seal, the above method further comprises removing at least one of the seals.

In another embodiment, there is provided a kit comprising a plurality of process challenge devices of any one of the embodiments of the process challenge device described herein.

In another embodiment, there is provided a kit comprising a plurality of process challenge devices selected from a plurality of the embodiments of the process challenge devices described herein. In one embodiment, for example, the plurality of process challenge devices may include different heat-transfer modulating bodies, different space volumes, different sterilant accesses, different quantities of sterilant accesses, different number of containers, or combinations thereof.

The above summary of the present invention is not intended to describe each disclosed embodiment or every implementation of the present invention. The description that follows more particularly exemplifies illustrative embodiments.

DEFINITIONS

The terms "flexible wall" or "flexible walls" refers to a wall or walls which can be sufficiently deformed to allow at least a 5 percent, preferably at least a 10 percent, change in volume of a space defined by the wall or walls. The change in volume may result from a change in pressure outside of the space defined by the wall.

The term "heat-transfer modulating body" refers to a body which controls the time required to raise the temperature of an indicator adjacent the body to the sterilization process temperature. For example, where a steam sterilization process temperature is 132 °C, the heat-transfer modulating body increases the time required for the indicator to reach 132 °C by slowing the rate at which heat is transferred to the indicator from, for example, a sterilization chamber.

The term "surround" refers to a heat-transfer modulating body or walls of the body positioned at least partially around the indicator but not completely enclosing the indicator.

The terms "envelop" or "enveloping" refer to a heat-transfer modulating body or walls of the body positioned to completely enclose the indicator.

The term "impervious to the sterilant" refers to walls that do not allow sterilant to pass through, except where an opening is provided to allow sterilant to enter any space defined by the walls. For example, the walls may be comprised of a continuous material which is not porous to the sterilant.

The term "pervious to the sterilant" refers to a heat-transfer modulating body or a wall or walls of the body that allow sterilant to pass through the body or the walls. For example, the body or walls may be comprised of a material which is porous to the sterilant, and/or the body or walls may include a plurality of openings or spaces through which the sterilant may pass.

The term "comprising" and variations thereof (e.g., comprises, includes, etc.) do not have a limiting meaning where these terms appear in the description and claims.

As used herein, "a," "an," "the," "at least one," and "one or more" are used interchangeably, unless the context clearly dictates otherwise.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

Also herein, the recitations of numerical ranges by endpoints include all numbers subsumed within that range (e.g., a volume of 5 to 1000 cm$^3$ includes a volume of 5, 63, 75.5, 1000 cm$^3$ etc.).

BRIEF DESCRIPTIONS OF THE DRAWINGS

FIG. 1 is a perspective view of one embodiment of a device according to the present invention, which includes an opening for the sterilant access.

FIG. 2 is a perspective view of another embodiment of a device according to the present invention, which includes a heat-transfer modulating body and a duct for a sterilant access.

FIG. 3 is a perspective view of one embodiment of a duct for a sterilant access.

FIG. 4 is a perspective view of another embodiment of a duct for a sterilant access.

FIG. 5 is a perspective view of another embodiment of a duct for a sterilant access.

FIG. 6 is a perspective view of another embodiment of a device according to the present invention, which includes a heat-transfer modulating body and an opening for a sterilant access.

FIG. 7A is a perspective view of another embodiment of a device according to the present invention, which includes a heat-transfer modulating body, an opening for a sterilant access, and a duct in fluid communication with the opening.

FIG. 7B is a partial schematic cross-section of the device of FIG. 7A further illustrating the opening and the duct.

FIG. 8 is a perspective view of another embodiment of a device according to the present invention, which includes a heat-transfer modulating body and an opening for a sterilant access.

FIG. 9A is a perspective view of another embodiment of a device according to the present invention, which includes a heat-transfer modulating body, a first container within a second container, and openings for a sterilant access in the first and second containers.

FIG. 9B is a schematic cross-section view of the device of FIG. 9A.

FIG. 10 is a perspective view of another embodiment of a device according to the present invention, which includes a heat-transfer modulating body with walls impervious to a sterilant and an opening for a sterilant access.

FIG. 11 is a perspective view of another embodiment of a device according to the present invention, which includes a heat-transfer modulating body and a valve for a sterilant access.

FIG. 12 is a perspective view of another embodiment of a device according to the present invention, which includes a heat-transfer modulating body and two valves for a sterilant access and for exiting a gas from the device, respectively.

FIG. 13 is a perspective view of the two valves in FIG. 12.

FIG. 14 is a perspective view of another embodiment of a device according to the present invention, which includes a heat-transfer modulating body, an opening for a sterilant access with a removable seal to seal the opening, and a duct for a second sterilant access.

FIG. 15 is a perspective view of another embodiment of a device according to the present invention, which includes a process indicator and a duct for a sterilant access.

FIG. 16 is a perspective view of another embodiment of a device according to the present invention, which includes a heat-transfer modulating body and two openings for sterilant accesses.

FIG. 17 is a perspective view of another embodiment of a device according to the present invention, which includes a heat-transfer modulating body, an opening for a sterilant access, and an absorbent sheet. FIG. 18 is a perspective view of another embodiment of a device according to the present invention, which includes a heat-transfer modulating body, an opening for a sterilant access covered with a permeable vent material, and an absorbent sheet.

## DETAILED DESCRIPTION OF ILLUSTRATIVE EMBODIMENTS OF THE INVENTION

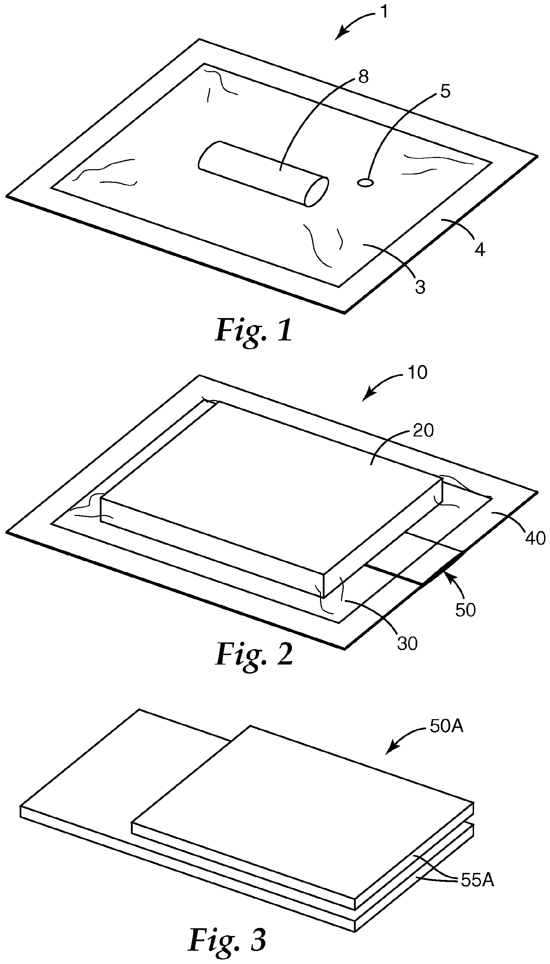

Process challenge device 1 illustrated in FIG. 1 is one embodiment of a process challenge device described herein. Container 3 is constructed with two sheets, each a flexible material and each forming a wall, heat sealed together at seal 4 to define the space in which process indicator 8 resides. For certain embodiments, the space within container 3 contains a volume of gas, typically determined at atmospheric pressure, of at least 5 cubic centimeters. For certain embodiments, the volume of gas is not more than 100 cubic centimeters. Although not shown, additional sheets could be used to form additional walls, for example, one or more gussets. Additional sheets could also be used together with the two sheets to add additional layers to the walls. Examples of suitable flexible materials include films of nylon, polyester, polyethylene, polypropylene, foil, and the like, and combinations thereof. For certain embodiments, the films are laminate films, examples of which include nylon/polyethylene/foil/polyethylene laminate films, clear polyester/polypropylene laminate films, and the like.

For certain embodiments, including any one of the device, method, and kit embodiments described herein, all of the walls of the first container are flexible walls.

For certain embodiments, including any one of the device, method, and kit embodiments described herein which include a second container, all of the walls of the second container are flexible walls.

For certain embodiments, including any one of the device, method, and kit embodiments described herein which include at least one additional container, all of the walls of the at least one additional container are flexible walls.

For certain embodiments, including any one of the device, method, and kit embodiments described herein, the flexible walls are laminate film.

Device 1 also includes opening 5 as a sterilant access in one of the two walls. For certain embodiments, the area of opening 5 is at least 0.1, 0.2, or 0.5 mm$^2$, and for certain embodiments, opening 5 has an area of not more than 100, 50, 20, or 10 cm$^2$. The size of opening 5 can be selected for a selected sterilization process. For example, for a sterilization process which includes vacuum cycles, the opening 5 may be relatively small. For certain embodiments, the area of opening 5 is at least 0.5 mm$^2$ and not more than 20 cm$^2$. In another example, for a gravity sterilization process without vacuum cycles, the opening 5 may be relatively large. For certain embodiments, the area of opening 5 is at least 10 cm$^2$ and not more than 100 cm$^2$. Device 1 is shown with one opening 5. However, more than one opening can be included. The openings can each have the same area or different areas. Some or all of the openings can be sealed with a removable seal, and one or more of the seals can selected and removed for using device 1 in a selected sterilization process.

Device 1 is illustrated with both walls being a flexible material, although in other embodiments one or more of the walls can be a rigid material as long as at least one wall is flexible. Because at least one wall of device 1 is flexible, relatively thin and low cost materials can be used for the wall or walls. Moreover, in certain embodiments, the flexible material allows the volume of the space defined by the walls to vary as a pressure differential varies between the space and outside the space or container. Thus, when the pressure outside of the container is increase, such as when a sterilant under pressure is applied to the container, the volume of the space decreases. This may increase the resistance of the device to a sterilant by resisting or slowing entrance of the sterilant into the space. Furthermore, when the pressure outside of the container is reduced, such as when applying a vacuum to the device to remove air or another gas from the space to facilitate displacement of the air or gas by the sterilant, the volume of the container increases. This may increase the resistance of the device as well by resisting or slowing exiting of the air or gas from the space.

Process indicator 8 or any process indicator referred to in any of the embodiments described herein can be one or more indicators and one or more types of indicators, for example, a biological indicator (BI) and/or a chemical indicator (CI).

Process challenge device 10 illustrated in FIG. 2 is another embodiment of a process challenge device described herein. Device 10 includes container 30 constructed with two sheets, each a flexible material and each forming a wall, heat sealed together at seal 40 to define the space in which heat-transfer modulating body 20 resides. Heat-transfer modulating body 20 envelops a process indicator such as described in FIG. 1 for indicator 8. Because heat-transfer modulating body 20 envelops the indicator, at least a portion of the body or at least a portion of walls comprising the body are porous. In one example, the body 20 is an ATTEST Rapid 5 Test Pack Plus #41382 (available form 3M Company, St. Paul, MN) This envelops an ATTEST 1292 Rapid Biological Indicator and COMPLY STERIGAGE 1243 Steam Chemical Integrator (all available from 3M Company, St. Paul, Minnesota). For certain embodiments, the space within container 30 contains a volume of gas, typically determined at atmospheric pressure, of at least 5 cubic centimeters. For certain embodiments, the volume of gas is not more than 100 cubic centimeters. Although not shown, additional sheets could be used to form additional walls, for example, one or more gussets. Additional sheets could also be used together with the two sheets to add additional layers to the walls. Examples of suitable sheet material are described above in reference to FIG. 1.

Device 10 also includes duct 50 as a sterilant access entering through seal 40 and extending to heat-transfer modulating body 20. Duct 50 comprises at least two layers of sheet material, each having an area and at least one major surface adjacent to a major surface of another of the at least two layers, each major surface comprising at least a portion of the area. FIGS. 3, 4, and 5 illustrate some embodiments of duct 50 that may be used in device 10 or any of the embodiments of a device described herein which include a duct. Duct 50A illustrated in FIG. 3 includes two layers of sheet material 55A, each having a major surface adjacent to a major surface of the other layer. Any sterilant entering the space within container 30 or any gas and/or liquid exiting the space travels through the area where the major surfaces are adjacent each other. For certain embodiments, the major surfaces adjacent to each other have an area of at least 10 mm$^2$ where the major surfaces are adjacent to each other. For certain embodiments, the area is at least 10 cm$^2$. For certain embodiments, the major surfaces adjacent to each other have an area of not more than 100 cm$^2$ where the major surfaces are adjacent to each other.

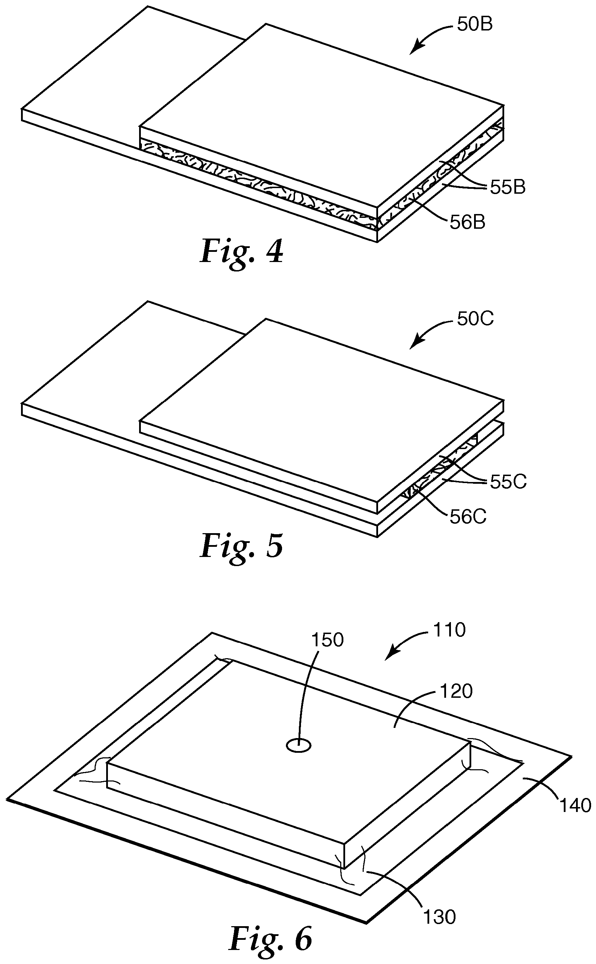

Duct 50B illustrated in FIG. 4 further includes a porous spacer 56B between the layers of sheet material 55B. Examples of the porous spacer include, for example, woven, knit, and nonwoven fabrics or mats, particles or beads held in place on the adjacent surfaces of the sheet material or on an additional sheet material, protrusions on the surfaces of the sheet material or on an additional sheet material, and the like. For certain embodiments, the porous spacer is removable. For certain embodiments, the porous spacer is a nonwoven fabric or mat. The presence of porous spacer 56B may allow sterilant to pass through duct 50B more easily than through duct 50A shown in FIG. 3, and thereby reduce the resistance of a device for use in a selected a sterilization process.

Duct 50C illustrated in FIG. 5 includes a porous spacer 56C which is narrower than the width of the layers of sheet material 55C. The amount by which porous spacer 56C is narrower than the width of the layers of sheet material 55C may further increase the ease of a sterilant to pass through duct 50C, and thereby reduce the resistance of a device for use in a selected sterilization process.

Process challenge device 110 illustrated in FIG. 6 is another embodiment of a process challenge device described herein. Device 110 includes container 130 constructed with two sheets, each a flexible material and each forming a wall, heat sealed together at seal 140 to define the space in which heat-transfer modulating body 120 resides. Heat-transfer modulating body 120 envelops a process indicator such as described in FIG. 1 for indicator 8 or as described in FIG. 2 for heat-transfer modulating body 20. For certain embodiments, the space within container 130 contains a volume of gas, typically determined at atmospheric pressure, of at least 5 cubic centimeters. For certain embodiments, the volume of gas is not more than 100 cubic centimeters. Although not shown, additional sheets could be used to form additional walls, for example, one or more gussets. Additional sheets could also be used together with the two sheets to add additional layers to the walls. Examples of suitable sheet material are described above in reference to FIG. 1.

Device 110 also includes opening 150 as a sterilant access in one of the two walls. For certain embodiments, the area of opening 150 is at least 0.5 mm$^2$, and for certain embodiments, opening 150 has an area of not more than 100 cm$^2$. The size of opening 150 can be selected for a selected sterilization process. For example, for a sterilization process which includes vacuum cycles, the opening 150 may be relatively small. For certain embodiments, the area of opening 150 is at least 0.5 mm$^2$ and not more than 20 cm$^2$. In another example, for a gravity sterilization process without vacuum cycles, the opening 150 may be relatively large. For certain embodiments, the area of opening 150 is at least 10 cm$^2$ and not more than 100 cm$^2$. Device 110 is shown with one opening 150. However, more than one opening can be included. The openings can each have the same area or different areas. Some or all of the openings can be sealed with a removable seal, and one or more of the seals can selected and removed for using device 110 in a selected sterilization process.

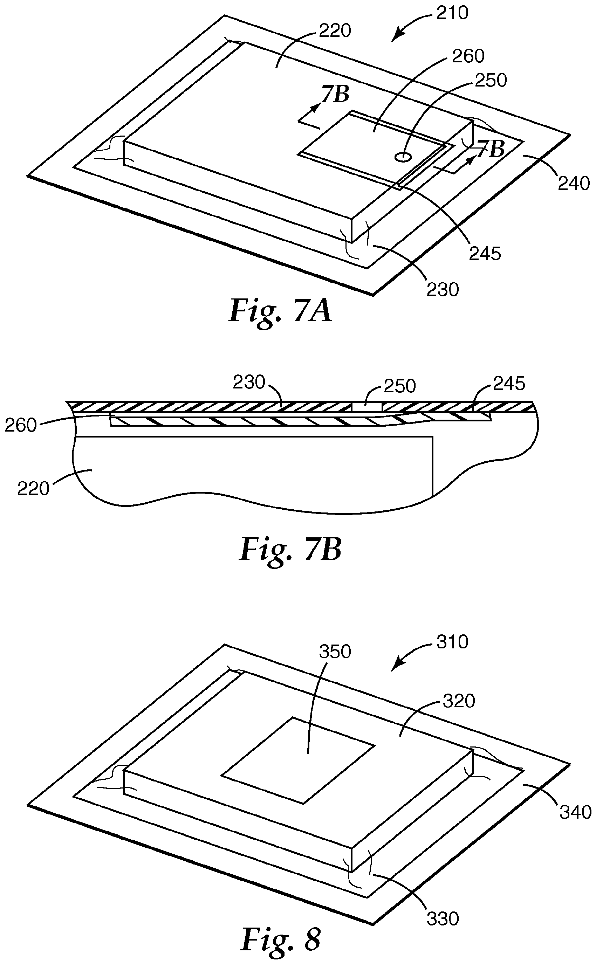

Process challenge device 210 illustrated in FIGS. 7A and 7B is another embodiment of a process challenge device described herein. Device 210 is similar to device 110 illustrated in FIG. 6 with container 230 constructed with two sheets, each a flexible material and each forming a wall, heat sealed together at seal 240 to define the space in which heat-transfer modulating body 220 resides, and with opening 250 as a sterilant access in one of the two walls. Device 210 further includes duct 260 through which a sterilant may enter container 230 after entering opening 250. Duct 260 is constructed by sealing at seals 245 a sheet material to the inside of the wall with the opening 250. Duct 260 may provide a more consistent resistance to entrance of a sterilant or exiting of a gas as the heat-transfer modulating body is modified or various body designs, materials, and surfaces are selected for a selected sterilization process.

Process challenge device 310 illustrated in FIG. 8 is another embodiment of a process challenge device described herein. Device 310 is similar to device 110 illustrated in FIG. 6 with container 330 constructed with two sheets, each a flexible material and each forming a wall, heat sealed together at seal 340 to define the space in which heat-transfer modulating body 320 resides. Opening 350 is a sterilant access in one of the two walls and is larger than the opening 150 illustrated in FIG. 6. Opening 350 may be selected for a selected process as discussed above for device 1. In one example, the selected process can be a gravity sterilization process which does not use a vacuum cycle. For certain embodiments, the area of opening 350 is at least 10 cm$^2$ and not more than 100 cm$^2$.

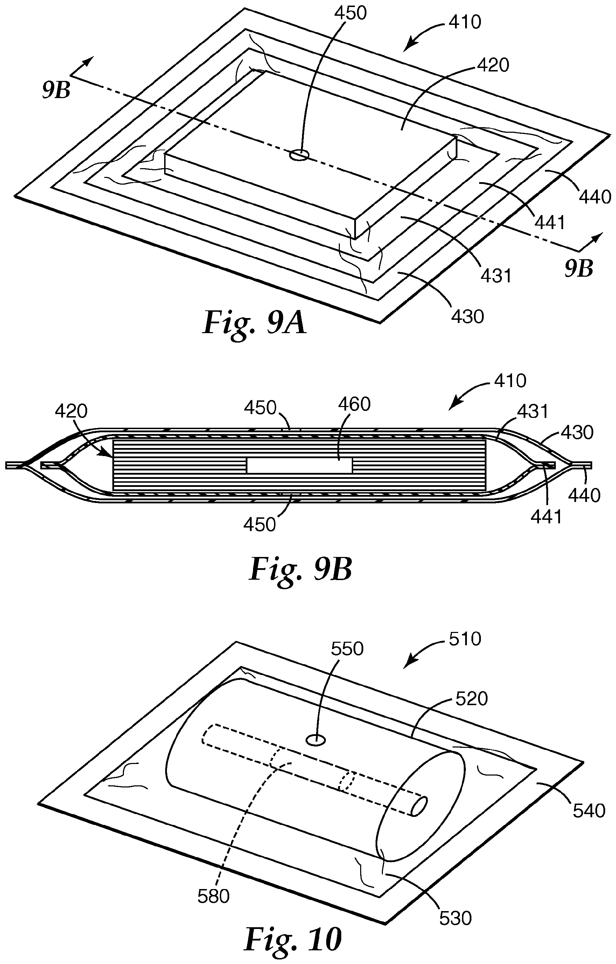

Process challenge device 410 illustrated in FIGS. 9A and 9B is another embodiment of a process challenge device described herein. Device 410 is similar to device 110 illustrated in FIG. 6 with first container 431 constructed with two sheets, each a flexible material and each forming a wall, heat sealed together at seal 441 to define the space in which heat-transfer modulating body 420 resides, and with opening 450 a sterilant access in one of the two walls. Device 410 further includes second container 430 also constructed with two sheets, each a flexible material and each forming a wall, heat sealed together at seal 440 to define a second space in which first container 431 resides. Second container 430 also includes opening 450 on the opposite side of opening 450 in first container 431 as illustrated in FIG. 9B. The cross-section of device 410 in FIG. 9B also illustrates a cross-section of heat-transfer modulating body 420 illustrating space 460 for a process indicator (not shown) and layers of material comprising walls of the heat-transfer modulating body which envelop space 460 and an indicator therein. The design of heat-transfer modulating body 420 illustrated in FIG. 9B may provide unexpected resistance to entrance of a sterilant into space 460 when used in the present flexible container, such as containers 430 and 431. When pressure is increased outside of container 430 and 431, such as when a sterilant under pressure is applied to the containers, the containers reduce in volume, compressing body 420, and thereby increasing the resistance of the device to a sterilant.

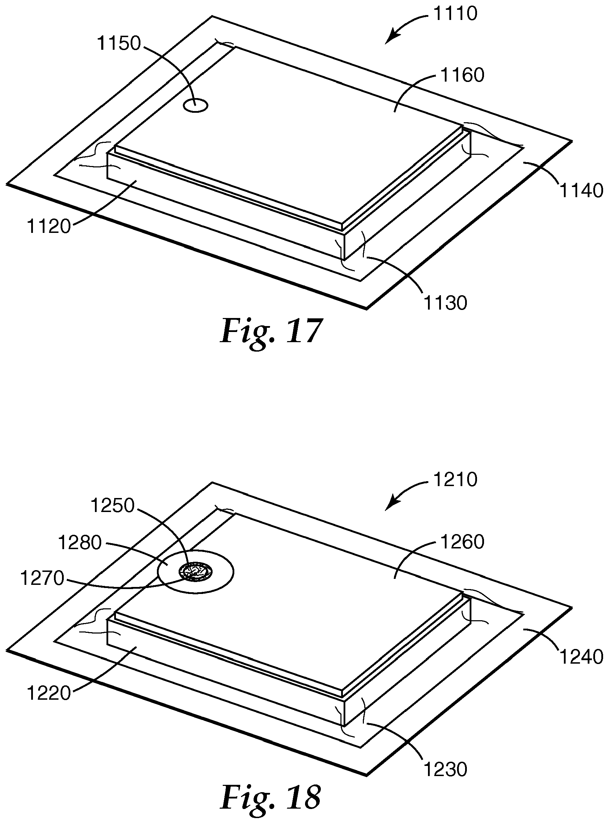

Although not shown, process challenge device 410 may include an absorbent material as described and illustrated below in reference to FIGS. 17 and 18. The absorbent material may be positioned between containers 430 and 431 and adjacent openings 450. The absorbent material may also be positioned between heat-transfer modulating body 420 and the wall of container 431 with opening 450.

Process challenge device 510 illustrated in FIG. 10 is another embodiment of a process challenge device described herein. Device 510 is similar to device 110 illustrated in FIG. 6 with first container 530 constructed with two sheets, each a flexible material and each forming a wall, heat sealed together at seal 540 to define the space in which heat-transfer modulating body 520 resides, and with opening 550 a sterilant access in one of the two walls. Heat-transfer modulating body 520, however, is a solid body which surrounds but does not envelop process indicator 580. Except for the opening in body 520 through which indicator 580 is inserted, body 520 may be impervious to a sterilant. Body 520 may be selected for a selected sterilization process with the walls of body 520 being thicker and/or having a lower thermal diffusivity for greater resistance or thinner and/or having a higher thermal diffusivity for lesser resistance to a sterilization process.

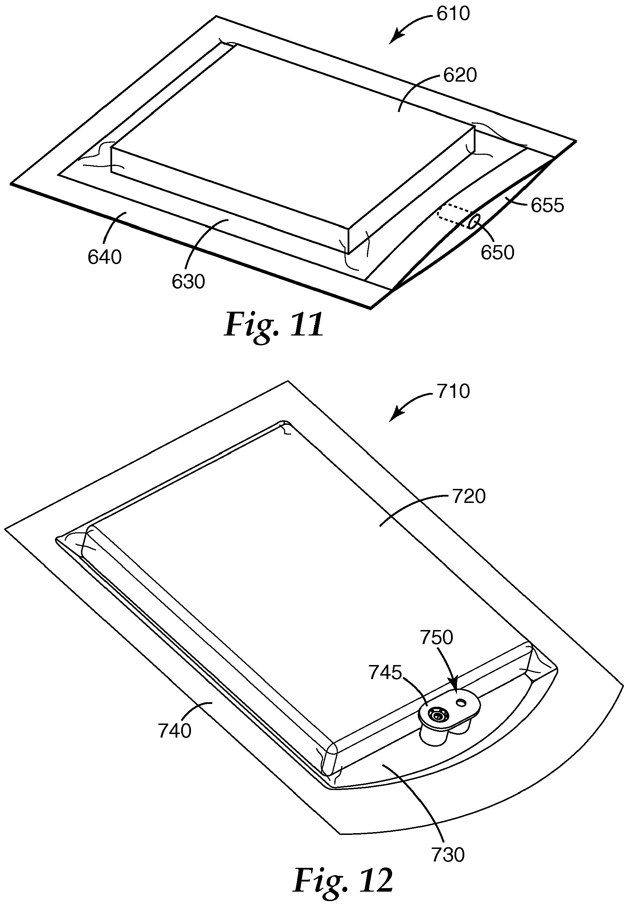

Process challenge device 610 illustrated in FIG. 11 is another embodiment of a process challenge device described herein. Device 610 is similar to device 110 illustrated in FIG. 6 with first container 630 constructed with two sheets, each a flexible material and each forming a wall, heat sealed together at seal 640 to define the space in which heat-transfer modulating body 620 resides. Device 610 includes pressure-actuating valve 650 as a sterilant access mounted in housing 655 sealed to the two sheets. Device 610 is illustrated with one valve, but more than one valve may be used. For certain embodiments, the at least one pressure-actuating valve is a combination valve. For certain embodiments, the at least one pressure-actuating valve is actuated when there is a pressure difference between space within the container and outside of the container. For certain embodiments, the pressure difference is at least 6.895 kPa (1 psi). For certain embodiments, the pressure difference is not more than 345 kPa (50 psi). For certain embodiments, the sterilant access comprises at least two pressure-actuating valves. For certain embodiments, at least one pressure-actuating valve is actuated when the pressure is higher outside of the container than in the space within the container. For certain embodiments, the pressure is higher by at least 6.895 kPa (1 psi). For certain embodiments, the pressure is higher by not more than 345 kPa (50 psi).

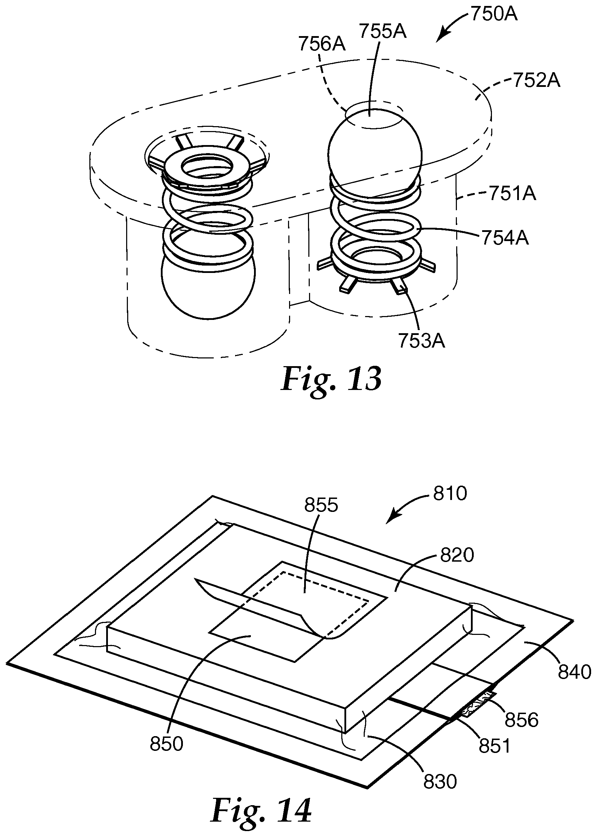

Process challenge device 710 illustrated in FIG. 12 is another embodiment of a process challenge device described herein. Device 710 is similar to device 110 illustrated in FIG. 6 with first container 730 constructed with two sheets, each a flexible material and each forming a wall, heat sealed together at seal 740 to define the space in which heat-transfer modulating body 720 resides. Device 710 includes pressure-actuating double valve 750 as a sterilant access sealed to one of the walls with seal 745. Valves 750 are positioned so that a sterilant enters the space within container 730 through one of the valves and a gas and/or liquid exits the space within container 730 through the other valve. Valve 750 is further illustrated in FIG. 13 as valve 750A. Seal 745 seals the sheet material of the wall to valve housing flange 752A. Ball 755A, spring 754A, and spring retainer 753A are positioned within valve housing 751A. A gas and/or liquid passes through opening 75 6A in valve housing 751A when the valve is actuated.

Process challenge device 810 illustrated in FIG. 14 is another embodiment of a process challenge device described herein. Device 810 is similar to both devices 310 illustrated in FIG. 8 and 10 illustrated in Figure 2. Container 830 is constructed with two sheets, each a flexible material and each forming a wall, heat sealed together at seal 840 to define the space in which heat-transfer modulating body 820 resides. Opening 850 is a sterilant access in one of the two walls and is sealed with a removable seal 855.

Device 810 also includes duct 851 as a sterilant access entering through seal 840 and extending to heat-transfer modulating body 820. Duct 851 comprises at least two layers of sheet material, each having an area and at least one major surface adjacent to a major surface of another of the at least two layers, each major surface comprising at least a portion of the area. Duct 851 is illustrated as duct 50C illustrated in FIG. 5 except that porous spacer 856 protrudes from duct 851 sufficiently for ease of removing porous spacer 856 for use in a selected sterilization process. Any of the embodiments of a duct described in reference to FIG. 2 may be used.

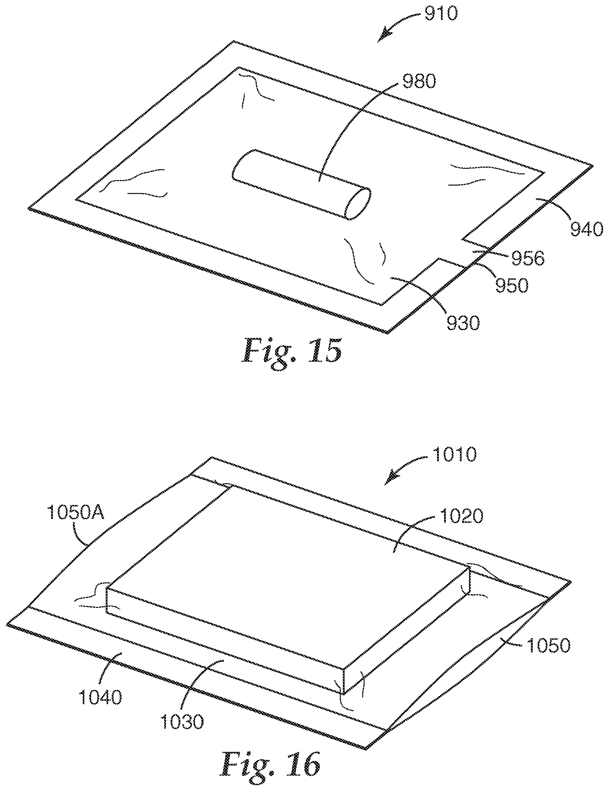

Process challenge device 910 illustrated in FIG. 15 is another embodiment of a process challenge device described herein. Device 910 is similar to device 1 illustrated in FIG. 1 with container 930 constructed with two sheets, each a flexible material and each forming a wall, heat sealed together at seal 940 to define the space in which process indicator 980 resides. Duct 950 is a sterilant access in an edge of container 930. Duct 950 is constructed by leaving sheet material portion 956 unsealed. Here the at least two layers of sheet material comprising duct 950 are an extension of the walls formed by the flexible material.

Process challenge device 1010 illustrated in FIG. 16 is another embodiment of a process challenge device described herein. Device 1010 includes container 1030 constructed with two sheets, each a flexible material and each forming a wall, heat sealed together at seal 1040 to define the space in which heat-transfer modulating body 1020 resides. Openings 1050 and 1050A are sterilant accesses. The area of openings 1050 and 1050A can be conveniently determined by not sealing the flexible material at each end of the device as illustrated or by sealing a portion of the flexible material at each end of the device.

Process challenge device 1110 illustrated in FIG. 17 is another embodiment of a process challenge device described herein. Device 1110 includes container 1130 constructed with two sheets, each a flexible material and each forming a wall, heat sealed together at seal 1140 to define the space in which heat-transfer modulating body 1120 resides. Heat-transfer modulating body 1120 envelops a process indicator as described above for FIG. 6. For certain embodiments, the space within container 1130 contains a volume of gas as described above for FIG. 6. Although not shown, additional sheets could be used to form additional walls, for example, one or more gussets. Additional sheets could also be used together with the two sheets to add additional layers to the walls. Examples of suitable flexible material are described above in reference to FIG. 1.

Device 1110 also includes opening 1150 as a sterilant access in one of the two walls. For certain embodiments, the area of opening 1150 is as described above for FIG. 6. Device 1110 is shown with one opening 1150. However, more than one opening can be included. The openings can each have the same area or different areas. Some or all of the openings can be sealed with a removable seal, and one or more of the seals can selected and removed for using device 1110 in a selected sterilization process.

Device 1110 also includes absorbent material 1160 for absorbing a condensate, such as a condensate of a sterilant, for example, a condensate of steam. Absorbent material 1160 is shown as a sheet material adjacent opening 1150. However, forms other than sheets of absorbent material may be used. Examples of absorbent materials that may be used include any one of or a combination of fibers, webs, films, microporous films, membranes, absorbents, foams, powders, gums, polymeric gels, microparticles, nanoparticles, nonwoven materials, including spunbond materials, knitted materials, meltblown materials, and composite materials. Suitable materials include any one or a combination of cellulosic fibers; cotton; glass; rayon; nylon; synthetic fibers such as polyvinyl alcohol, polyvinyl chloride, polybutylene terephthalate, polytetrafluoroethylene, polypropylene, polyethylene, polylactic acid, polyester, and polyurethane; wood pulp; acrylics; olefin; wool; paper; metal; superabsorbent polymers; superabsorbent particles; cheesecloth; polymeric gels and hydrogels such as copolymers of polyvinylpyrrolidone and any of the polymeric gels disclosed in U.S. Pat No. 6,352,837 (Witcher) which are incorporated herein by reference.

Process challenge device 1210 illustrated in FIG. 18 is another embodiment of a process challenge device described herein. Device 1210 includes container 1230 constructed with two sheets, each a flexible material and each forming a wall, heat sealed together at seal 1240 to define the space in which heat-transfer modulating body 1220 resides. Heat-transfer modulating body 1220 envelops a process indicator as described above for FIG. 6. For certain embodiments, the space within container 1230 contains a volume of gas as described above for FIG. 6. Although not shown, additional sheets could be used to form additional walls, for example, one or more gussets. Additional sheets could also be used together with the two sheets to add additional layers to the walls. Examples of suitable flexible material are described above in reference to FIG. 1.

Device 1210 also includes opening 1250 as a sterilant access in one of the two walls. For certain embodiments, the area of opening 1250 is as described above for FIG. 6. Device 1210 is shown with one opening 1250. However, more than one opening can be included. The openings can each have the same area or different areas. Some or all of the openings can be sealed with a removable seal, and one or more of the seals can selected and removed for using device 1210 in a selected sterilization process.

Device 1210 also includes absorbent material 1260 for absorbing a condensate, such as a condensate of a sterilant, for example, a condensate of steam. Absorbent material 1260 is shown as a sheet material adjacent opening 1250. Absorbent materials as described above for Device 1110 may be used.

Device 1210 also includes permeable vent material 1270 covering opening 1250. The vent material is permeable to the sterilant. Preferably, the vent material is also permeable to any gas, such as air, within container 1230. Suitable permeable vent materials include porous webs, microporous films, porous membranes, at least partially reticulated foams, woven materials, nonwoven materials, including spunbond materials, knitted materials, meltblown materials, and composite materials.

The permeable vent material increases resistance of the device to the sterilant, for example, by slowing the rate of sterilant entering the container. For certain embodiments, including any one of the embodiments described herein which includes an opening for a sterilant access, the opening is covered with a permeable vent material.

Device 1210 also includes stiffening material 1280. The stiffening material reduces or prevents wrinkling of the flexible material which makes up container 1230 at opening 1250, during a sterilization cycle. This maintains consistency of the opening size during a sterilization cycle, thereby increasing consistency of the challenge provided by the device. Stiffening material 1280 in device 1210 is attached to container 1230 by a pressure sensitive adhesive which also serves to retain permeable vent material 1270 in position covering opening 1250. Suitable stiffening materials include films, including polymeric films, metal sheeting, including foils, papers, card boards, polymer coatings, nonwovens, gels, and combinations thereof. The stiffening material may be attached to the area around the sterilant access opening by an adhesive, including a pressure sensitive adhesive, thermal bonding, or the like.

For certain embodiments, including any one of the embodiments described herein which includes an opening for a sterilant access, the opening is at least partially surrounded with a stiffening material. For certain of these embodiments, the opening is fully surrounded with stiffening material, for example, as illustrated by stiffening material 1280 in FIG. 18.

For certain embodiments, including any one of the embodiments described herein, an absorbent material which absorbs sterilant condensate is included within the process challenge device container. The absorbent material may be any one or combination of those described above. When more than one container is included in the device, for example, as shown in FIGS. 9A and 9B, the absorbent material may be included within any one of the containers, all of the containers, or a portion of the containers. For certain embodiments, preferably the absorbent material is positioned as described for FIG. 9B and as shown in FIGS 17 and 18.

For certain embodiments, including any one of the embodiments described herein which includes an absorbent material, the absorbent material is a layer of absorbent material or sheet material. Sheet materials include nonwovens, knits, wovens, papers, card stock, cardboard, porous membranes, porous films, and the like. For certain of these embodiments, the sheet material is a nonwoven or a paper towel. For certain of these embodiments, preferably the layer of absorbent material or the sheet material has a thickness of at least 0.25 mm, preferably at least 0.4 mm, more preferably at least 1 mm. For certain of these embodiments, the layer of absorbent material or the sheet material has a thickness of not more than 10 mm, preferably not more than 7.5 mm, more preferably not more than 5.5 mm. For certain of these embodiments, the thickness is 1 mm to 7.5 mm. For certain of these embodiments, the thickness is 2 mm to 5.5 mm.

For certain embodiments, including any one of the embodiments described herein which includes an absorbent material, the absorbent material is positioned adjacent a sterilant access. The sterilant access may be an opening, a duct, a pressure-actuating valve, or a combination thereof. For certain of these embodiments, preferably the sterilant access is an opening. When positioned adjacent a sterilant access at least a portion of sterilant entering the container passes by and/or through the absorbent material. For certain of these embodiments, the absorbent material is positioned adjacent a sterilant access such that all of the sterilant entering the container passes by and/or through the absorbent material.

The layer of absorbent material or the absorbent sheet material covers a sufficient area and is present in sufficient amount to prevent sterilant condensate from sealing off the sterilant access in an uncontrolled or non-reproducible manner during a sterilization cycle.

Incorporation of the absorbent material adjacent a sterilant access, such as opening 1150 in FIG. 17 and opening 1250 in FIG. 18, has now been found to make the operation of the process challenge device more consistent and prevent inadvertent sealing of the opening from entrance of the sterilant, caused by formation of condensate within the container.

For certain embodiments, the walls comprising any one of the embodiments of a heat-transfer modulating body described herein have a thickness of at least 0.3 cm. The walls may include one, two, three, or more layers. The heat-transfer modulating body can be adjusted for wall thickness by removing one or more layers, and thereby decrease the resistance of the device to sterilization conditions. Also, one or more additional layers can be added to increase the wall thickness of the heat-transfer modulating body, and thereby increase the resistance of the device to sterilization conditions. Moreover, the layers can have the same or different thermal diffusivities, allowing the thermal diffusivity of the heat-transfer modulating body to be adjusted for a particular sterilization process. For certain embodiments, preferably the walls of the heat-transfer modulating body have a thermal diffusivity ($\alpha$) of not more than $1 \times 10^{-5}$ m$^2$/s at 20 °C.

The process challenge devices described herein can be provided without or with one or more process indicators. The indicator or indicators are chosen to be used with sterilization conditions to be employed in a particular sterilization process. When the device is provided without the indicator, the indicator is selected and placed in the device prior to using the device in the sterilization process. For example, for a steam sterilization process, a steam sterilization indicator is selected for the indicator. Moreover, the indicator can be chosen based upon the amount of exposure to sterilization conditions required to cause the indicator to indicate that the exposure has occurred. The choice of the sterilization indicator can thereby by used to increase or decrease the resistance of the sterilization process challenge device.

When absorbent material is present, the space within the container is dimensioned to allow the indicator and absorbent material to fit within the space. The absorbent material can absorb the condensate of a sterilant to prevent or reduce the amount of condensate that can contact the sterilization indicator, thereby preventing undesired indicator error. Furthermore, preventing condensate formation on the indicator reduces the heat gain of the indicator caused by heat transfer from the sterilant. For example, with steam sterilization, the absorbent material absorbs water which would otherwise condense on the indicator. A suitable absorbent material is cellulose or other absorbent fiber, such as absorbent paper.

The absorbent material can extend beyond the ends of the indicator, such that when placed within a space within a container, the indicator can be retrieved from the space by pulling on the absorbent material.

For certain embodiments, when an indicator is within a space within a container, the distance between indicator and the walls defining the space is preferably less than 5 cm. For certain embodiments the distance is less than 2 cm, 1 cm, 0.75 cm, or 0.5 cm. For certain embodiments, the indicator can contact the walls. Preferably, the distance between the indicator and the walls is sufficient to allow a layer of absorbent material between the walls and the indicator.

The process challenge device of the present invention can be provided without or with an indicator, which is chosen to be used with sterilization conditions to be employed in a particular sterilization process. As indicted above, the indicator can be a BI, a CI, a combination thereof. A plurality of indicators can also be used in the process challenge device. When the device is provided without an indicator, an indicator is selected and placed in the device prior to using the device in a sterilization process. The indicator or indicators can be covered with a porous material, such as paper or fabric. For certain embodiments, the indicator is sandwiched between or wrapped in two or more layers of a porous material. For certain embodiments, preferably the porous material absorbs sterilant condensate.

As indicated above, for certain embodiments, the space within the container contains a volume of gas of at least 5 cm$^3$. For certain embodiments, including any one of the above embodiments of the device described herein, the volume of gas contained within the space is at least 10, 25, or 50 cm$^3$. For certain of these embodiments, the volume of gas contained within the space is not more 1000 cm$^3$, 500 cm$^3$, 250 cm$^3$, 125 cm$^3$, or 75 cm$^3$. Because the containers described herein include at least one flexible wall, the above volumes are typically determined at atmospheric pressure.

As indicated above, for certain embodiments, the walls comprising any one of the heat-transfer modulating bodies described herein have a thickness of at least 0.3 cm. For certain embodiments, including any one of the embodiments of the device described herein, the thickness is preferably at least about 0.5 cm. For certain of these embodiments, the thickness is at least 0.6 cm, 0.75 cm, 1 cm, 1.25 cm, or 2.5 cm. For certain embodiments, the thickness is at most 10 cm or 5 cm.

For certain embodiments, the walls comprising any one of the heat-transfer modulating bodies described herein have a thermal diffusivity ($\alpha$) of not more than $1 \times 10^{-5}$ m$^2$/s at 20 °C. Suitable materials for the walls of the body include, for example, stainless steel ($\alpha=0.405 \times 10^{-5}$ m$^2$/s), polypropylene, DELRIN, nylon ($\alpha=1.3 \times 10^{-7}$ m$^2$/s), polyester, polycarbonate, polytetrafluoroethylene ($\alpha=1.1 \times 10^{-7}$ m$^2$/s), and the like. For certain of these embodiments, the thermal diffusivity is not more than $5 \times 10^{-7}$ m$^2$/s at 20 °C. For certain of these embodiments, the thermal diffusivity is not more than $2 \times 10^{-7}$ m$^2$/s at 20 °C. The thermal diffusivity of the material indicates how rapidly the material adjusts its temperature to that of its surroundings. For example, a material with a relatively low thermal diffusivity heats up more slowly than a material with a higher thermal diffusivity in an environment at an elevated temperature, such as a sterilization chamber. Thermal diffusivity is used in heat transfer analysis and is the ratio of thermal conductivity to volumetric heat capacity as follows:

$$\alpha = \kappa/\rho C_p$$

where $\kappa$ is thermal conductivity (W/mK), $\rho$ is density (kg/m$^3$), and $C_p$ is specific heat capacity (J/kgK). Thus, using these parameters, a suitable material or combination of materials for the walls of the heat-transfer modulating body can be chosen to achieve a desired resistance to sterilization conditions used in a sterilization process. For example, a material with a particular thermal diffusivity can be used for the walls of the heat-transfer modulating body, or the walls of the heat-transfer modulating body can be comprised of two or more layers, where at least two of the layers have different thermal diffusivities, to provide walls with a composite thermal diffusivity.

Sterilization indicators which can be used in the process challenge device described herein are known and include biological indicators and chemical indicators. Examples of biological indicators include ATTEST 1292 Rapid Biological Indicators (available from 3M Company, St. Paul, MN) and those described in U.S. Patent No.

6,623,955 can be used. Examples of chemical indicators include COMPLY STERIGAGE 1243 Steam Chemical Integrator (available from 3M Company) and those described in U.S. Patent No. 5,916,816 can be used.

## Claims

1. A sterilization process challenge device (410) comprising:

   a first container (431) comprising walls which define a first space within the first container (431);
   a process indicator within the first space;
   at least one sterilant access for a sterilant to enter the first space within the first container (431);
   wherein at least one of the walls which defines the first space (460) is a flexible wall; and wherein the walls are impervious to the sterilant;

   **characterized by**

   a second container (430) comprising:

   walls which define a second space within the second container (430); and
   at least one sterilant access (450) for a sterilant to enter the second space;

   wherein the first container (431) is within the second space; wherein at least one of the walls which defines the second space is a flexible wall; wherein the walls which define the second space are impervious to the sterilant; and wherein the second space has a volume which can vary as a pressure differential varies between the second space and outside of the second container (430).

2. The device of claim 1, further comprising a heat-transfer modulating body (420) adjacent the indicator.

3. The device of claim 1 or claim 2, wherein the first space (460) has a volume which can vary as a pressure differential varies between the first space (460) and outside of the first container (431).

4. The device of any one of claims 1 through 3, wherein the first space further contains a volume of gas at atmospheric pressure of at least 5 cubic centimeters.

5. The device of any one of claims 1 through 4, wherein the at least one sterilant access comprises at least one opening (150) in at least one of the walls which defines the first space, wherein the at least one opening (150) has an area.

6. The device of any one of claims 1 through 4, wherein the at least one sterilant access comprises at least one duct (150) comprising at least two layers of sheet material, each having an area and at least one major surface adjacent to a major surface of another of the at least two layers, each major surface comprising at least a portion of the area.

7. The device of any one of claims 1 through 4, wherein the at least one sterilant access comprises at least one pressure actuating valve (650; 750).

8. The device of claim 5, wherein the at least one opening is at least partially surrounded with a stiffening material (1280).

9. The device of claim 5 or claim 8, wherein the at least one opening is covered with a permeable vent material (1270).

10. The device of any one of claims 1 through 9, further comprising an absorbent material (1260) which absorbs sterilant condensate within the first container.

11. The device of claim 10, wherein the absorbent material (1260) is a sheet material adjacent the at least one sterilant access.

12. The device of claim 11, wherein the sheet material has a thickness of at least 0.25 mm and not more than 10 mm.

13. A method of determining the effectiveness of a sterilization process, the method comprising:

    providing a process challenge device of any one of claims 1 through 12;
    positioning the process challenge device in a sterilization chamber;
    exposing the process challenge device to a sterilant at an elevated temperature; and
    determining whether or not the process indicator indicates that it has been exposed to sterilization process conditions effective for sterilizing an article.

## Patentansprüche

1. Sterilisationsprozess-Challengevorrichtung (410), umfassend:

   einen ersten Behälter (431), der Wände umfasst, die einen ersten Raum innerhalb des ersten Behälters (431) bestimmen;
   einen Prozessindikator innerhalb des ersten Raums;
   mindestens einen Sterilisationsmittelzugang für den Eintritt eines Sterilisationsmittels in den ersten Raum innerhalb des ersten Behälters (431); wobei mindestens eine der Wände, die den ersten Raum (460) bestimmt, eine flexible Wand ist; und wobei die Wände für das Sterilisationsmittel undurchlässig sind;
**gekennzeichnet durch**
einen zweiten Behälter (430), der umfasst:

    Wände, die einen zweiten Raum innerhalb des zweiten Behälters (430) bestimmen; und
    mindestens einen Sterilisationsmittelzugang (450) für den Eintritt eines Sterilisationsmittels in den zweiten Raum; wobei sich der erste Behälter (431) innerhalb des zweiten Raums befindet; wobei mindestens eine der Wände, die den zweiten Raum bestimmt, eine flexible Wand ist; wobei die Wände, die den zweiten Raum bestimmen, für das Sterilisationsmittel undurchlässig sind; und wobei der zweite Raum ein Volumen aufweist, das variieren kann, wenn eine Druckdifferenz zwischen dem zweiten Raum und außerhalb des zweiten Behälters (430) variiert.

2. Vorrichtung nach Anspruch 1, ferner umfassend einen Wärmeübertragungsmodulationskörper (420) angrenzend an den Indikator.

3. Vorrichtung nach Anspruch 1 oder Anspruch 2, wobei der erste Raum (460) ein Volumen aufweist, das variieren kann, wenn eine Druckdifferenz zwischen dem ersten Raum (460) und außerhalb des ersten Behälters (431) variiert.

4. Vorrichtung nach einem der Ansprüche 1 bis 3, wobei der erste Raum ferner ein Gasvolumen bei Atmosphärendruck von mindestens 5 Kubikzentimetern enthält.

5. Vorrichtung nach einem der Ansprüche 1 bis 4, wobei der mindestens eine Sterilisationsmittelzugang mindestens eine Öffnung (150) in mindestens einer der Wände, die den ersten Raum bestimmt, umfasst, wobei die mindestens eine Öffnung (150) eine Fläche aufweist.

6. Vorrichtung nach einem der Ansprüche 1 bis 4, wobei der mindestens eine Sterilisationsmittelzugang mindestens einen Kanal (150) umfasst, der mindestens zwei Schichten von Lagenmaterial umfasst, die jeweils eine Fläche und mindestens eine Hauptoberfläche angrenzend an eine Hauptoberfläche einer anderen der mindestens zwei Schichten aufweisen, wobei jede Hauptoberfläche mindestens einen Abschnitt der Fläche umfasst.

7. Vorrichtung nach einem der Ansprüche 1 bis 4, wobei der mindestens eine Sterilisationsmittelzugang mindestens ein Druckbetätigungsventil (650; 750) umfasst.

8. Vorrichtung nach Anspruch 5, wobei die mindestens eine Öffnung mindestens teilweise von einem Versteifungsmaterial (1280) umgeben ist.

9. Vorrichtung nach Anspruch 5 oder Anspruch 8, wobei die mindestens eine Öffnung mit einem durchlässigen Entlüftungsmaterial (1270) bedeckt ist.

10. Vorrichtung nach einem der Ansprüche 1 bis 9, ferner umfassend ein Absorptionsmaterial (1260), das Sterilisationsmittelkondensat innerhalb des ersten Behälters absorbiert.

11. Vorrichtung nach Anspruch 10, wobei das Absorptionsmaterial (1260) ein Lagenmaterial ist, das an den mindestens einen Sterilisationsmittelzugang angrenzt.

12. Vorrichtung nach Anspruch 11, wobei das Lagenmaterial eine Dicke von mindestens 0,25 mm und nicht mehr als 10 mm aufweist.

13. Verfahren zum Bestimmen der Wirksamkeit eines Sterilisationsverfahrens, wobei das Verfahren Folgendes umfasst:

    Bereitstellen einer Prozess-Challengevorrichtung nach einem der Ansprüche 1 bis 12;
    Positionieren der Prozess-Challengevorrichtung in einer Sterilisationskammer;
    Aussetzen der Prozess-Challengevorrichtung einem Sterilisationsmittel bei erhöhter Temperatur; und Bestimmen, ob der Prozessindikator anzeigt, dass er Bedingungen eines Sterilisationsverfahrens ausgesetzt worden ist, die zum Sterilisieren eines Gegenstands wirksam sind.

**Revendications**

1. Dispositif d'épreuve de procédé de stérilisation (410) comprenant :

    un premier récipient (431) comprenant des parois qui définissent un premier espace au sein du premier récipient (431) ;
    un indicateur de procédé au sein du premier espace ;
    au moins un accès de stérilisant permettant à un stérilisant de pénétrer dans le premier espace au sein du premier récipient (431) ;
    dans lequel au moins l'une des parois qui définit le premier espace (460) est une paroi

flexible ; et dans lequel les parois sont imperméables au stérilisant ;

**caractérisé par**

un deuxième récipient (430) comprenant :

des parois qui définissent un deuxième espace au sein du deuxième récipient (430) ; et au moins un accès de stérilisant (450) permettant à un stérilisant de pénétrer dans le deuxième espace ; dans lequel le premier récipient (431) se trouve au sein du deuxième espace ; dans lequel au moins l'une des parois qui définit le deuxième espace est une paroi flexible ; dans lequel les parois qui définissent le deuxième espace sont imperméables au stérilisant ; et dans lequel le deuxième espace a un volume qui peut varier à mesure qu'un différentiel de pression varie entre le deuxième espace et l'extérieur du deuxième récipient (430).

2. Dispositif selon la revendication 1, comprenant en outre un corps de modulation de transfert de chaleur (420) adjacent à l'indicateur.

3. Dispositif selon la revendication 1 ou la revendication 2, dans lequel le premier espace (460) a un volume qui peut varier à mesure qu'un différentiel de pression varie entre le premier espace (460) et l'extérieur du premier récipient (431).

4. Dispositif selon l'une quelconque des revendications 1 à 3, dans lequel le premier espace contient en outre un volume de gaz à pression atmosphérique d'au moins 5 centimètres cubes.

5. Dispositif selon l'une quelconque des revendications 1 à 4, dans lequel ledit au moins un accès de stérilisant comprend au moins une ouverture (150) dans au moins une des parois, qui définit le premier espace, dans lequel ladite au moins une ouverture (150) a une aire.

6. Dispositif selon l'une quelconque des revendications 1 à 4, dans lequel ledit au moins un accès de stérilisant comprend au moins un conduit (150) comprenant au moins deux couches de matériau en feuille, ayant chacune une aire et au moins une surface principale adjacente à une surface principale d'une autre desdites au moins deux couches, chaque surface principale comprenant au moins une partie de l'aire.

7. Dispositif selon l'une quelconque des revendications 1 à 4, dans lequel ledit au moins un accès de stérilisant comprend au moins une soupape d'actionnement de pression (650 ; 750).

8. Dispositif selon la revendication 5, dans lequel ladite au moins une ouverture est au moins partiellement entourée d'un matériau de raidissement (1280).

9. Dispositif selon la revendication 5 ou la revendication 8, dans lequel ladite au moins une ouverture est couverte d'un matériau de ventilation perméable (1270).

10. Dispositif selon l'une quelconque des revendications 1 à 9, comprenant en outre un matériau absorbant (1260) qui absorbe un condensat de stérilisant au sein du premier récipient.

11. Dispositif selon la revendication 10, dans lequel le matériau absorbant (1260) est un matériau en feuille adjacent audit au moins un accès de stérilisant.

12. Dispositif selon la revendication 11, dans lequel le matériau en feuille a une épaisseur d'au moins 0,25 mm et de pas plus de 10 mm.

13. Procédé de détermination de l'efficacité d'un procédé de stérilisation, le procédé comprenant :

la fourniture d'un dispositif d'épreuve de procédé selon l'une quelconque des revendications 1 à 12 ; le positionnement du dispositif d'épreuve de procédé dans une chambre de stérilisation ; l'exposition du dispositif d'épreuve de procédé à un stérilisant à une température élevée ; et le fait de déterminer si, oui ou non, l'indicateur de procédé indique qu'il a été exposé à des conditions de procédé de stérilisation efficaces pour stériliser un article

*Fig. 1*

*Fig. 2*

*Fig. 3*

*Fig. 4*

*Fig. 5*

*Fig. 6*

**Fig. 7A**

**Fig. 7B**

**Fig. 8**

**Fig. 9A**

**Fig. 9B**

**Fig. 10**

*Fig. 11*

*Fig. 12*

*Fig. 13*

*Fig. 14*

*Fig. 15*

*Fig. 16*

**Fig. 17**

**Fig. 18**

**EP 2 362 786 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2001006610 A1
- WO 2009137442 A1
- US 2002022246 A1
- US 6352837 B, Witcher
- US 6623955 B
- US 5916816 A